(19) Europäisches Patentamt / European Patent Office / Office européen des brevets

(11) **EP 4 310 072 A1**

(12) **EUROPEAN PATENT APPLICATION**
published in accordance with Art. 153(4) EPC


(43) Date of publication:
**24.01.2024 Bulletin 2024/04**

(21) Application number: **22914729.3**

(22) Date of filing: **27.12.2022**

(51) International Patent Classification (IPC):
***C07C 229/52*** (2006.01) ***D06M 13/372*** (2006.01)
***C08K 5/18*** (2006.01) ***A61K 8/41*** (2006.01)
***A61Q 17/04*** (2006.01)

(86) International application number:
**PCT/CN2022/142177**

(87) International publication number:
**WO 2023/125475 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2021 CN 202111628792**



(71) Applicant: **MFCI (Huanggang) Co., Ltd.**
**Huanggang, Hubei 438011 (CN)**

(72) Inventors:
- **LIU, Jianjun**
  **huanggang, Hubei 438011 (CN)**
- **XU, Wenli**
  **huanggang, Hubei 438011 (CN)**
- **ZENG, Cheng**
  **huanggang, Hubei 438011 (CN)**
- **WU, Wei**
  **huanggang, Hubei 438011 (CN)**
- **XIONG, Yaodong**
  **Huanggang, Hubei 438011 (CN)**

(74) Representative: **karo IP**
**karo IP Patentanwälte**
**Kahlhöfer Rößler Kreuels PartG mbB**
**Postfach 32 01 02**
**40416 Düsseldorf (DE)**


(54) **ULTRAVIOLET ABSORBER, COMPOSITION, COSMETIC PRODUCT, AND PROCESS FOR PREPARING COSMETIC PRODUCT**


(57) The present disclosure discloses an ultraviolet absorber composition and a cosmetic product. The ultraviolet absorber is diethylamino hydroxybenzoyl hexyl benzoate; a maximum particle size $d_{(0.9)}$ of the ultraviolet absorber is within a range of 50-100 μm; moreover, a width difference between a minimum particle size $d_{(0.1)}$ and a maximum particle size $d_{(0.9)}$ of the ultraviolet absorber is within a range of 30-100 μm; and the ultraviolet absorber has an effective span of not greater than 3 as determined by an instrument.


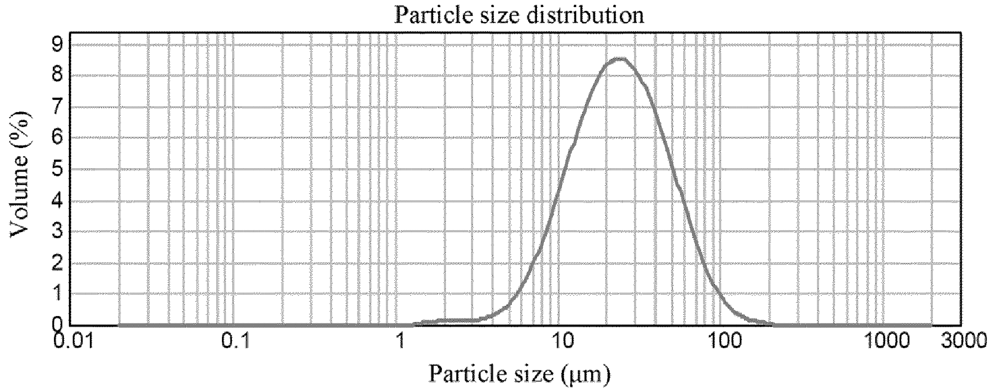


FIG. 1

EP 4 310 072 A1

## Description

## TECHNICAL FIELD

The present disclosure belongs to the field of ultraviolet protection, and more specifically, relates to an ultraviolet absorber and a composition and use thereof. The use may be preparation of a cosmetic product using the ultraviolet absorber or the composition.

## BACKGROUND

2-(4-N,N-diethylamino-2-hydroxybenzoyl) hexyl benzoate (Diethylamino hydroxybenzoyl hexyl benzoate, DHHB) is a typical and commonly used oil-soluble organic ultraviolet absorber and has an oil dissolution temperature of not higher than 50°C and a sun protection band of 320-400 nm. In particular, as an ultraviolet A (UVA) absorber for absorbing near ultraviolet rays, DHHB can prevent ultraviolet penetration by chemically absorbing energy of ultraviolet rays and converting the energy into heat energy, thus effectively delaying the phenomena of light aging and degradation of products made from polymer materials such as plastics and rubber during use. When used as an additive in cosmetic products such as sunscreen creams, DHHB can effectively prevent the skin from being affected by ultraviolet rays.

Taking use of DHHB in cosmetic products (in China, European Union and Australia, the maximum allowable concentration is 10%) as an example, oil phases and water phases are usually separately treated for conventional oil/water (O/W) and water/oil (W/O) system sunscreen creams using traditional preparation processes of sunscreen cosmetic products, and ultraviolet absorbers, such as DHHB, are usually dissolved in the oil phases. Taking preparation of the W/O system sunscreen creams as an example, specifically, oil phase raw materials (including DHHB) are put into an emulsifying pot, stirred and heated to 80-85°C to ensure that all the raw materials are completely dissolved or evenly dispersed, followed by heat preservation for 10 min. Water phase raw materials are put into a water phase pot, stirred and heated to 80-85°C to ensure that all the raw materials are completely dissolved or evenly dispersed, followed by heat preservation for 10 min. Vacuum and stirring functions of the emulsifying pot are enabled, the water phase raw materials are pumped into the emulsifying pot (namely, including oil phase components) at a certain rate for homogenization, and after the homogenization is completed, all the raw materials are mixed, stirred and cooled to about 45°C. Active substances, volatile ingredients, flavors and preservatives are added into the emulsifying pot and evenly stirred (for homogenization). Products are discharged and filled after passing inspections. For example, in a known patent document 1, an oily substance is introduced into a mixing container, and an ultraviolet (UV) filter is preferably stirred at 85-95°C. Alternatively, for example, in a patent document 2, when an oil phase system is prepared, diethylhexyl butamido triazone, DHHB and bis-ethylhexyloxyphenol methoxyphenyl triazine are mixed and added into an oil phase solvent for dissolution at 80-85°C to obtain an oil phase mixture.

During early synthesis of DHHB, a pink crude product prepared from a solution in a crystal form is purified by chromatography, then a solvent is removed by distillation, and finally, a clean final product is bottled and sold as a melt. In a later use process, a whole package needs to be heated to a temperature higher than a melting point of DHHB, so that a liquid product can be taken out from the package. With development of use technologies, the bottled DHHB gradually has defects during use. Based on demands, a more convenient novel crystallization preparation method for DHHB particles is developed, and granular DHHB with a particle size in millimeters can be directly prepared. In addition, it is well-known that the typical oil-soluble organic ultraviolet absorber has an oil dissolution temperature of not higher than 50°C. Therefore, producers general do not need to highly consider adverse effects of the particle size on DHHB.

As described in the traditional methods, the oil phases and the water phases are separately heated in advance, and then the two phases are mixed, stirred and cooled to room temperature. A lot of time or energy is consumed when the oil phases and the water phases are heated to 70°C or above, and meanwhile, longer time or an auxiliary means, such as water cooling, is required for rapid cooling of the phases during cooling. Moreover, the flavors and unstable components are added in extremely limited time in the process. Cosmetic companies need to consider how to reduce energy consumption and carbon emissions not only in terms of raw materials, transportation and packaging, but also in product processes. Based on these situations, a low-energy emulsification (LEE) method is usually used for carrying out product processing at present. For example, in a so-called cold method, namely, implementing at room temperature of 20-25°C, in the patent document 1, a solvent and any other conventional auxiliary substances are converted into a final sunscreen preparation.

However, in actual production, the low-energy emulsification method is limited to a certain extent, because the granular DHHB prepared by a novel crystallization technology will have limitations on technological processing during use. The first most typical problem is that the oil dissolution time of DHHB is prolonged in doubles due to the decrease of temperature or the increase of concentration. The second problem is that the stability and rhythmicity of the whole production technology are affected. When restricted by too many factors, a production process is prone to fluctuation, thereby seriously affecting the stability of a production technology, seriously affecting the stability of a production rhythm,

and further affecting the quality of products.

Patent document 1: CN105358221B, disclosed on August 23, 2019, REAGENT CONTAINING LARGE NUMBER OF UV STABILIZERS.
Patent document 2: CN109908020A, disclosed on June 21, 2019, SUNSCREEN COMPOSITION, PREPARATION METHOD AND USE THEREOF.

## SUMMARY

### 1. Problems to be solved

A first object of the present disclosure is to provide an ultraviolet absorber or a composition based on the same suitable for both the traditional methods and a low temperature (room temperature 20-25°C) processing method.
A second object of the present disclosure is to solve the problems that existing ultraviolet absorbers or compositions based on the same have serious adverse effects on the stability of a production technology and the stability of a production rhythm when used in the traditional processes and the low temperature (room temperature 20-25°C) processing method. The ultraviolet absorber has a maximum particle size of less than 100 μm.

### 2. Technical schemes

In order to solve the above problems, the present disclosure provides an ultraviolet absorber. The ultraviolet absorber is 2-(4-N,N-diethylamino-2-hydroxybenzoyl) hexyl benzoate, which specifically has a structural formula as follows:

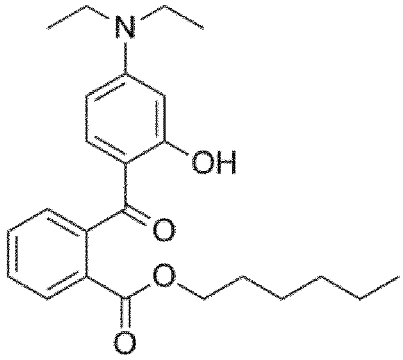

the ultraviolet absorber has a median particle size $d_{(0.5)}$ of less than 100 μm;
the maximum particle size $d_{(0.9)}$ of the ultraviolet absorber is within a range of 50-100 μm; and,
a width difference between the minimum particle size $d_{(0.1)}$ and the maximum particle size $d_{(0.9)}$ of the ultraviolet absorber is within a range of 30-100 μm.

It is to be noted herein that the applicant has found through experiments that when the ultraviolet absorber has an average particle size of less than 100 μm, the dissolution rate and the dissolution time of the ultraviolet absorber have sharp decrease intervals. When particles are broken to a nanometer level or a sub-micron level, not only is the precision of a preparation technology required to be improved, thereby increasing the grinding cost and energy consumption, but also nanoparticles attract each other in a dispersion process to further form large particles in a dispersion medium due to a micro-action force between the nanoparticles, thereby having a possibility of decreasing the dissolution rate.
In terms of range, the numeral value of the maximum particle size $d_{(0.9)}$ may be any value selected from any one of the following value ranges: 50-100 μm, 50-90 μm, 50-80 μm, 50-70 μm, 50-60 μm, 60-100 μm, 60-90 μm, 60-80 μm, 60-70 μm, 70-100 μm, 70-90 μm, 70-80 μm, 80-100 μm, 80-90 μm, and 90-100 μm; and the numeral value of the width difference between the minimum particle size $d_{(0.1)}$ and the maximum particle size $d_{(0.9)}$ may be any value selected from any one of the following value ranges: 30-100 μm, 30-90 μm, 30-80 μm, 30-70 μm, 30-60 μm, 30-50 μm, 30-40 μm, 40-100 μm, 40-90 μm, 40-80 μm, 40-70 μm, 40-60 μm, 40-50 μm, 50-100 μm, 50-90 μm, 50-80 μm, 50-70 μm, 50-60 μm, 60-100 μm, 60-90 μm, 60-80 μm, 60-70 μm, 70-100 μm, 70-90 μm, 70-80 μm, 80-100 μm, 80-90 μm, and 90-100 μm.
Further, the minimum particle size $d_{(0.1)}$ of the ultraviolet absorber is within a range of 5-15 μm. In terms of

range, the numeral value of the minimum particle size $d_{(0.1)}$ may be any value selected from any one of the following value ranges: 5-15 μm, 5-12 μm, 5-10 μm, 5-8 μm, 5-6 μm, 7-15 μm, 7-12 μm, 7-10 μm, 7-8 μm, 9-15 μm, 9-12 μm, 9-10 μm, 10-15 μm, 10-12 μm, 12-15 μm, and 14-15 μm.

Further, the median particle size $d_{(0.5)}$ of the ultraviolet absorber is within a range of 20-40 μm. In terms of range, the numeral value of the median particle size $d_{(0.5)}$ may be any value selected from any one of the following value ranges: 20-40 μm, 20-35 μm, 20-30 μm, 20-25 μm, 25-40 μm, 25-35 μm, 25-30 μm, 30-40 μm, 30-35 μm, and 35-40 μm.

Further, the ultraviolet absorber has an effective span of not greater than 3 as determined by an instrument. The numeral value of the effective span may be any value selected from any one of the following value ranges: 1-3, 1-2.5, 1-2, 1-1.5, 1.5-3, 1.5-2.5, 1.5-2, 2-3, 2-2.5, and 2.3-3.

Further, the ultraviolet absorber has a consistency within a range of 0.5-1.5 as determined by an instrument. The numeral value of the consistency may be any value selected from any one of the following value ranges: 0.5-1.5, 0.5-1.2, 0.5-1, 0.5-0.8, 0.5-0.6, 0.7-1.5, 0.7-1.2, 0.7-1, 0.7-0.8, 1-1.5, and 1-1.2.

Further, the maximum particle size $d_{(0.9)}$ of the ultraviolet absorber is within a range of 50-90 μm; and

the minimum particle size $d_{(0.1)}$ of the ultraviolet absorber is within a range of 5-15 μm; and
the median particle size $d_{(0.5)}$ of the ultraviolet absorber is within a range of 20-40 μm; and
the ultraviolet absorber has consistency of not greater than 1 as determined by an instrument.

Further, particle size distribution of the ultraviolet absorber meets the following requirements:

$$\alpha_{d5} = \frac{V_{d5}}{V_{d100}}$$

where

$\alpha_{d5}$ is a distribution coefficient of particles with a particle size of 5 μm;
$V_{d5}$ is a volume ratio of particles with a particle size of less than 5 μm in a particle group to the particle group, %;
$V_{d100}$ is a volume ratio of particles with a particle size of less than 100 μm in the particle group to the particle group, %;
and the distribution coefficient of small particle size particles of the particle group satisfies that the $\alpha_{d5}$ is equal to or less than 0.2.

Further, particle size distribution of the ultraviolet absorber meets the following requirements:

$$\alpha_{d10} = \frac{V_{d10}}{V_{d100}}$$

where

$\alpha_{d10}$ is a distribution coefficient of 10 μm particles;
$V_{d10}$ is a total volume ratio of particles with a particle size of less than 10 μm in the ultraviolet absorber to the ultraviolet absorber particles, %;
$V_{d100}$ is a total volume ratio of particles with a particle size of less than 100 μm in the ultraviolet absorber to the ultraviolet absorber particles, %;
and the distribution coefficient of small particle size particles of the ultraviolet absorber satisfies that $\alpha_{d10}$ is equal to or less than 0.2. It is to be noted herein that, the applicant has surprisingly found when the ultraviolet absorber contains too many small particles or even fine powders, the small particles are filled in spaces between large particles, thereby increasing contact points between the large particles and promoting agglomeration. However, when the volume of the small particles in the ultraviolet absorber is controlled within a certain range and differences between large particle size and small particle size are controlled, although the phenomenon of agglomeration is still present, the agglomeration degree is obviously reduced compared with that in the prior art. Furthermore, the differences between large particle size and small particle size are controlled, and the ultraviolet absorber has high agglomeration and looseness degrees. When a product of the present disclosure is put into technological use, the applicant has found that the product can be quickly dispersed by collision or simple mechanical stirring when poured into a container from a package, additional heating or other auxiliary treatment is not required, functional energy consumption is reduced, and process carbon emissions are reduced. Meanwhile, when the ultraviolet absorber of the present disclosure is usually dissolved in an organic phase for reprocessing, a liquid bridge force may be more easily

generated between the ultraviolet absorber and the organic phase due to a certain number of small particle size particles in absorption particles, and an adhesion force between interfaces is increased to a certain extent, so that the absorption particles are more easily and quickly distributed in the organic phase. Furthermore, the dissolution rate of the absorption particles with a small particle size is greater than that of the absorption particles with a large particle size, thereby further accelerating a dissolution process of the absorption particles.

It is worth further explaining that, in principle, any type of agitator may be used in a stirring process, such as a magnetic stirring core, an anchor agitator, a propeller agitator, an inclined plate agitator or a disk agitator. The size of an agitator relative to the volume of putted raw materials is not decisive. It is already known that the dissolution rate can be increased by increasing the speed of the agitator, namely revolution of the agitator/ minute. In the present disclosure, the agitator performs stirring at 100-600 rpm/min, preferably 200-500 rpm/min, and optimally 250-350 rpm/min.
Further, the distribution coefficient of small particle size particles of the ultraviolet absorber satisfies that $\alpha_{d10}$ is equal to or less than 0.15.
Further, the distribution coefficient of small particle size particles of the ultraviolet absorber satisfies that $\alpha_{d10}$ is equal to or greater than 0.05 and equal to or less than 0.15; in addition, the numeral value of the distribution coefficient of small particle size particles of the ultraviolet absorber may be any value selected from any one of the following value ranges: 0.05-0.15, 0.05-0.12, 0.05-0.1, 0.05-0.08, 0.05-0.06, 0.07- 0.15, 0.07- 0.12, 0.07-0.1, 0.09-0.15, 0.09-0.12, 0.09-0.1, 0.11-0.15, 0.11-0.12, and 0.12-0.15; and preferably, the distribution coefficient of small particle size particles satisfies that the $\alpha_{d10}$ is equal to or greater than 0.07 and equal to or less than 0.12. It is worth explaining that a certain number of small particle size particles are dispersed in gaps of large particle size particles, so that the ultraviolet absorption particles collide and are embedded into each other to achieve the effects of thorough depolymerization and dispersion, so as to achieve the effect of uniform mixing.
Further, $V_{d100}$ of the ultraviolet absorber is equal to or greater than 70%, preferably, equal to or greater than 85%, and further preferably, equal to or greater than 95%.
Further, under processing conditions, the dissolution time of the ultraviolet absorber in a solvent is not higher than 30 min;
the processing conditions are specifically as follows:

solvent: dibutyl adipate;
added concentration: 3-20 wt%;
temperature: 20-30°C;
stirring rate: 150±5 r/min;
and an index of the ultraviolet absorber dissolved in the solvent is that the solvent has a solid content of less than 0.5%.

Further, the dissolution time of the ultraviolet absorber in a solvent is not higher than 25 min.
A composition having an ultraviolet absorption effect includes an ultraviolet absorber, a dispersant, a film forming agent, an antioxidant and a lubricant; where the ultraviolet absorber includes the ultraviolet absorber according to any one of the above descriptions;
and at least one of the dispersant, the film forming agent, the antioxidant and the lubricant is an oil phase at a temperature of not lower than 20°C.
Further, the composition having an ultraviolet absorption effect includes 2-(4-N,N-diethylamino-2-hydroxybenzoyl) hexyl benzoate ultraviolet absorber particles according to any one of the above descriptions, dicaprylyl carbonate, dibutyl adipate, caprylic/capric triglyceride and tocopheryl acetate.
A cosmetic product includes the ultraviolet absorber or includes the composition as a raw material. The cosmetic product is defined in a broad meaning in the present disclosure, and the "cosmetic product" includes and is not limited to a whitening agent, a sunscreen cream, a sunscreen and the like.
A cosmetic product preparation process is characterized in that the ultraviolet absorber according to any one of the above descriptions or the composition according to any one of the above descriptions is used as a raw material, and when the raw material is dissolved in an oil phase:

the temperature is not higher than 50°C, preferably 40-50°C;
and the added concentration is not higher than 20 wt%, preferably 3-20 wt%.

The present disclosure further provides use of the ultraviolet absorption particles as a sunscreen component of a cosmetic product, or as an ultraviolet protection additive component for fabric surface treatment, or as an ultraviolet protection additive component for a polymer material. A final product prepared has a high ultraviolet absorption coverage rate and a good ultraviolet absorption effect.
In addition, as different consumers have large differences in skin characteristics, sensitivity and absorption

properties, the consumers have also put forward higher requirements for sunscreen cosmetic products, and different groups of consumers often need differentiated products adapted to their own skin characteristics. With differentiation of consumer demands, the consumers have more and more diversified demands for product categories, product types and product characteristics of cosmetic products. Thus, enterprises need to produce or process sunscreen cosmetic products of different categories, types or characteristics, thereby greatly increasing the production and processing complexity of cosmetic products of cosmetic enterprises. For example, the production categories or types of products need to be adjusted frequently. Meanwhile, while the demands for the product categories are gradually increasing, the consumers have also paid more and more attention to the quality of the cosmetic products, especially have paid attention to the uniformity of properties of the cosmetic products of same categories and same types. However, as the production and processing of the cosmetic products have the characteristics of long production flow, complexity in production and processing, multiple operating units and the like, these problems are to be solved urgently during production of the cosmetic products, but have not been solved yet. Moreover, corresponding technical persons are constantly trying to improve processing methods or apparatuses to solve the problems. During improvement of the ultraviolet absorption particles, the applicant has further surprisingly found that the ultraviolet absorption particles of the present disclosure can greatly improve the stability of a production technology and can well maintain the rhythmicity of a production rhythm. When products of different models and different concentrations are produced, even when products of different categories and models are alternately produced, the stability of the production technology can still be maintained, so that the production technology has corresponding stability, the continuity of the production rhythm is maintained, and changes of the production stability are prevented from affecting the stability of product quality.

## BRIEF DESCRIPTION OF THE DRAWINGS

FIG. 1 shows a particle size distribution curve of a DHHB ultraviolet absorber in Example 1;
FIG. 2 shows a particle size distribution curve of a DHHB ultraviolet absorber in Example 2;
FIG. 3 shows a particle size distribution curve of a DHHB ultraviolet absorber in Example 3;
FIG. 4 shows a particle size distribution curve of a DHHB ultraviolet absorber in Example 4;
FIG. 5 shows a particle size distribution curve of a DHHB ultraviolet absorber in Example 5;
FIG. 6 shows a particle size distribution curve of a DHHB ultraviolet absorber in Example 6;
FIG. 7 is a photograph showing cold dissolution experiment results of a sample in Example 1 (shorter than 5 min);
FIG. 8 is a photograph showing cold dissolution experiment results of a sample in Comparative Example 1 (5 min);
FIG. 9 is a photograph showing cold dissolution experiment results of a sample in Comparative Example 1 (45 min);
FIG. 10 is a photograph showing cold dissolution experiment results of a sample in Comparative Example 1 (105 min);

and arrows in the figures point to undissolved DHHB samples.

## DETAILED DESCRIPTION

Unless otherwise defined, in the present disclosure, all technical terms and scientific terms used have the same meanings as those generally understood by persons skilled in the technical field to which the present disclosure belongs; and the term "and/or" as used herein includes any and all combinations of one or more related listed items.
In the present disclosure, those without specific conditions in embodiments are carried out according to conventional conditions or conditions suggested by manufacturers. All reagents or instruments used without manufacturers are conventional products that can be purchased on the market.
As used herein, the term "about" is used for providing the flexibility and imprecision associated with a provided term, a measure or a value. Persons skilled in the art can easily determine the degree of flexibility of specific variables. More specifically, although exemplary embodiments of the present disclosure have been described herein, the present disclosure is not limited to these embodiments, but includes any and all embodiments obtained by modifications, omissions, such as combinations of various embodiments, adaptive changes and/or substitutions that can be recognized by persons skilled in the art according to foregoing detailed descriptions. Definitions in the claims may be interpreted broadly in accordance with a language used in the claims and are not limited to embodiments described in the foregoing detailed descriptions or in implementation of the present application, and these embodiments shall be deemed as non-exclusive. Any steps enumerated in any method or process in the claims may be performed in any sequence and are not limited to the sequence stated in the claims. Therefore, the scope of the present disclosure shall be determined only by the attached claims and legal equivalents thereof and are not determined by instructions and embodiments provided above.
Unless otherwise defined, all technical terms and scientific terms used herein have the same meanings as those generally understood by persons of ordinary skill in the field to which the present disclosure belongs. In a case of

a contradiction, definitions in the specification shall prevail. When the mass, the concentration, the temperature, the time or other values or parameters is expressed in a range, a preferred range or a series of ranges defined by upper limit preferred values and lower limit preferred values, it shall be understood as specifically disclosing all ranges formed by any pairs of upper limits or preferred values in any ranges and lower limits or preferred values in any ranges, whether or not these ranges are separately disclosed. For example, the range of 1-50 shall be understood as including any values, value combinations, or subranges selected from 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 and all small values between the integers, such as 1.1, 1.2, 1.3, 1.4, 1.5, 1.6, 1.7, 1.8 and 1.9. In terms of subranges, the subranges are specifically considered as "nested subranges" that extend from any endpoint within a range. For example, the nested subranges of the exemplary range of 1-50 may include 1-10, 1-20, 1-30 and 1-40 in one direction, or 50-40, 50-30, 50-20 and 50-10 in another direction.

For example, the term "median particle size $d_{(0.5)}$" is used herein, where the "median particle size " is also called "middle particle size", and the "$d_{(0.5)}$ " may also be expressed as "Dv50" or "D50", indicating a corresponding particle size when a cumulative particle size distribution percentage (also referred to as an accumulative particle size distribution percentage) of a sample reaches 50%, and further, a physical meaning is that particles with a larger particle size account for 50%, and particles with a smaller particle size also account for 50%.

For example, the term "maximum particle size $d_{(0.9)}$" is used herein, where the "$d_{(0.9)}$ " may also be expressed as "Dv90" or "D90", indicating a corresponding particle size when a cumulative particle size distribution percentage of a sample reaches 90%, and a physical meaning is that particles with a larger particle size account for 10%, and particles with a smaller particle size account for 90%.

For example, the term "minimum particle size $d_{(0.1)}$" is used herein, where the "$d_{(0.1)}$ " may also be expressed as "Dv10" or "D10", indicating a corresponding particle size when a cumulative particle size distribution percentage of a sample reaches 10%, and a physical meaning is that particles with a larger particle size account for 90%, and particles with a smaller particle size account for 10%.

For example, the term "width difference between the minimum particle size $d_{(0.1)}$ and the maximum particle size $d_{(0.9)}$" used herein may also be called "effective particle size width" or "particle size width" or "particle size distribution width" or "effective particle width" or "particle width" or "particle distribution width", specifically indicating an absolute difference between a corresponding particle size when a cumulative particle size distribution percentage of a sample reaches a value $d_{(0.1)}$ and a corresponding particle size when a cumulative particle size distribution percentage of a sample reaches another value $d_{(0.9)}$.

**Particle size testing**

The particle size distribution and characteristics of the ultraviolet absorber described herein, such as the minimum particle size $d_{(0.1)}$, the $d_{(0.9)}$ and the consistency are determined by a laser diffraction method, and determination conditions are as follows:

particle size analyzer: Malvern MS2000 laser particle size analyzer;
name of an injector: Hydro 2000MU (A);
analysis mode: general;
particle size range: 0.02-2000 μm;
name of a dispersant: water.

**Example 1**

The ultraviolet absorber in this example is determined by a laser diffraction method, a Malvern MS2000 laser particle size analyzer is used as a particle size analyzer, and determination conditions and test results are as shown in Table 1 below.

**Table 1 Particle size determination data in Example 1**

| Refractive index of particles | Absorptivity of particles | Particle size range | Shading degree |
|---|---|---|---|
| 1.590 | 0 | 0.020-2000 μm | 13.60% |
| Name of a dispersant | Refractive index of a dispersant | Residual error | Concentration |
| Water | 1.330 | 1.617% | 0.0351%Vol |

(continued)

| Name of a dispersant | Refractive index of a dispersant | Residual error | Concentration |
|---|---|---|---|
| Span (β) | Consistency | Specific surface area | Surface area-average particle size D [3,2] |
| 2.008 | 0.638 | 0.331 $m^2/g$ | 18.113 μm |
| Volume-average particle size D[4,3] | $d_{(0.1)}$ | $d_{(0.5)}$ | $d_{(0.9)}$ |
| 29.840 μm | 9.647 μm | 23.866 μm | 57.575 μm |

Determination results show that the ultraviolet absorber in this example has particle size distribution characteristics as shown in Table 2 below, and a specific distribution curve is as shown in FIG. 1.

**Table 2 Particle size distribution table of an ultraviolet absorber in Example 1**

| Particle size (μm) | Volume within a range % | Particle size (μm) | Volume within a range % | Particle size (μm) | Volume within a range % |
|---|---|---|---|---|---|
| 0.724 | 0 | 5.754 | 0.75 | 45.709 | 5.76 |
| 0.832 | 0 | 6.607 | 1.17 | 52.481 | 4.88 |
| 0.955 | 0 | 7.586 | 1.73 | 60.256 | 3.95 |
| 1.096 | 0 | 8.710 | 2.44 | 69.183 | 3.03 |
| 1.259 | 0 | 10.000 | 3.26 | 79.433 | 2.20 |
| 1.445 | 0.01 | 11.482 | 4.17 | 91.201 | 1.48 |
| 1.660 | 0.07 | 13.183 | 5.09 | 104.713 | 0.94 |
| 1.905 | 0.10 | 15.136 | 5.96 | 120.226 | 0.55 |
| 2.188 | 0.11 | 17.378 | 6.70 | 138.038 | 0.31 |
| 2.512 | 0.12 | 19.953 | 7.27 | 158.489 | 0.17 |
| 2.884 | 0.12 | 22.909 | 7.60 | 181.970 | 0.10 |
| 3.311 | 0.13 | 26.303 | 7.69 | 208.930 | 0.03 |
| 3.802 | 0.18 | 30.200 | 7.53 | 239.883 | 0 |
| 4.365 | 0.28 | 34.674 | 7.13 | 275.423 | 0 |
| 5.012 | 0.46 | 39.811 | 6.53 | 316.228 | 0 |

The ultraviolet absorber in this example has an $\alpha_{d5}$ value of 0.0160, an $\alpha_{d10}$ value of 0.111, and a difference between D[3,2] and D[4,3] being 11.727 μm.

**Example 2**

The ultraviolet absorber in this example is determined by a laser diffraction method, a Malvern MS2000 laser particle size analyzer is used as a particle size analyzer, and determination conditions and test results are as shown in Table 3 below.

**Table 3 Particle size determination data in Example 2**

| Refractive index of particles | Absorptivity of particles | Particle size range | Shading degree |
|---|---|---|---|
| 1.590 | 0 | 0.020-2000 μm | 11.62% |
| Name of a dispersant | Refractive index of a dispersant | Residual error | Concentration |
| Water | 1.330 | 0.516% | 0.0308%Vol |
| Span (β) | Consistency | Specific surface area | Surface area-average particle size D [3,2] |
| 1.869 | 0.578 | 0.325 $m^2/g$ | 18.463 μm |

(continued)

| Volume-average particle size D[4,3] | $d_{(0.1)}$ | $d_{(0.5)}$ | $d_{(0.9)}$ |
|---|---|---|---|
| 27.328 μm | 9.862 μm | 22.470 μm | 51.867 μm |

Determination results show that the ultraviolet absorber in this example has particle size distribution characteristics as shown in Table 4 below, and a specific distribution curve is as shown in FIG. 2.

**Table 4 Particle size distribution table of an ultraviolet absorber in Example 2**

| Particle size (μm) | Volume within a range % | Particle size (μm) | Volume within a range % | Particle size (μm) | Volume within a range % |
|---|---|---|---|---|---|
| 0.724 | 0 | 5.754 | 0.66 | 45.709 | 5.42 |
| 0.832 | 0 | 6.607 | 1.14 | 52.481 | 4.46 |
| 0.955 | 0 | 7.586 | 1.79 | 60.256 | 3.51 |
| 1.096 | 0 | 8.710 | 2.62 | 69.183 | 2.60 |
| 1.259 | 0 | 10.000 | 3.59 | 79.433 | 1.78 |
| 1.445 | 0 | 11.482 | 4.66 | 91.201 | 1.08 |
| 1.660 | 0 | 13.183 | 5.71 | 104.713 | 0.56 |
| 1.905 | 0 | 15.136 | 6.69 | 120.226 | 0.13 |
| 2.188 | 0 | 17.378 | 7.47 | 138.038 | 0.01 |
| 2.512 | 0 | 19.953 | 8.00 | 158.489 | 0 |
| 2.884 | 0 | 22.909 | 8.22 | 181.970 | 0 |
| 3.311 | 0.02 | 26.303 | 8.12 | 208.930 | 0 |
| 3.802 | 0.07 | 30.200 | 7.74 | 239.883 | 0 |
| 4.365 | 0.15 | 34.674 | 7.12 | 275.423 | 0 |
| 5.012 | 0.35 | 39.811 | 6.33 | 316.228 | 0 |

The ultraviolet absorber in this example has an $\alpha_{d5}$ value of 0.006, an $\alpha_{d10}$ value of 0.104, and a difference between D[3,2] and D[4,3] being 8.865 μm.

**Example 3**

The ultraviolet absorber in this example is determined by a laser diffraction method, a Malvern MS2000 laser particle size analyzer is used as a particle size analyzer, and determination conditions and test results are as shown in Table 5 below.

**Table 5 Particle size determination data in Example 3**

| Refractive index of particles | Absorptivity of particles | Particle size range | Shading degree |
|---|---|---|---|
| 1.590 | 0 | 0.020-2000 μm | 16.97% |
| Name of a dispersant | Refractive index of a dispersant | Residual error | Concentration |
| Water | 1.330 | 0.508% | 0.0504%Vol |
| Span (β) | Consistency | Specific surface area | Surface area-average particle size D [3,2] |
| 2.230 | 0.691 | 0.295 $m^2/g$ | 20.356 μm |
| Volume-average particle size D[4,3] | $d_{(0.1)}$ | $d_{(0.5)}$ | $d_{(0.9)}$ |
| 37.899 μm | 9.803 μm | 30.410 μm | 77.626 μm |

Determination results show that the ultraviolet absorber in this example has particle size distribution characteristics as shown in Table 6 below, and a specific distribution curve is as shown in FIG. 3.

**Table 6 Particle size distribution table of an ultraviolet absorber in Example 3**

| Particle size (μm) | Volume within a range % | Particle size (μm) | Volume within a range % | Particle size (μm) | Volume within a range % |
|---|---|---|---|---|---|
| 0.724 | 0 | 5.754 | 0.87 | 45.709 | 6.40 |
| 0.832 | 0 | 6.607 | 1.22 | 52.481 | 6.20 |
| 0.955 | 0 | 7.586 | 1.65 | 60.256 | 5.83 |
| 1.096 | 0 | 8.710 | 2.14 | 69.183 | 5.27 |
| 1.259 | 0 | 10.000 | 2.68 | 79.433 | 4.52 |
| 1.445 | 0 | 11.482 | 3.25 | 91.201 | 3.64 |
| 1.660 | 0.01 | 13.183 | 3.80 | 104.713 | 2.69 |
| 1.905 | 0.09 | 15.136 | 4.34 | 120.226 | 1.75 |
| 2.188 | 0.10 | 17.378 | 4.83 | 138.038 | 0.97 |
| 2.512 | 0.11 | 19.953 | 5.27 | 158.489 | 0.25 |
| 2.884 | 0.13 | 22.909 | 5.64 | 181.970 | 0 |
| 3.311 | 0.18 | 26.303 | 5.95 | 208.930 | 0 |
| 3.802 | 0.26 | 30.200 | 6.19 | 239.883 | 0 |
| 4.365 | 0.39 | 34.674 | 6.36 | 275.423 | 0 |
| 5.012 | 0.59 | 39.811 | 6.44 | 316.228 | 0 |

The ultraviolet absorber in this example has an $\alpha_{d5}$ value of 0.019, an $\alpha_{d10}$ value of 0.107, and a difference between D[3,2] and D[4,3] being 17.543 μm.

**Example 4**

The ultraviolet absorber in this example is determined by a laser diffraction method, a Malvern MS2000 laser particle size analyzer is used as a particle size analyzer, and determination conditions and test results are as shown in Table 7 below.

**Table 7 Particle size determination data in Example 4**

| | | | |
|---|---|---|---|
| Refractive index of particles | Absorptivity of particles | Particle size range | Shading degree |
| 1.590 | 0 | 0.020-2000 μm | 12.22% |
| Name of a dispersant | Refractive index of a dispersant | Residual error | Concentration |
| Water | 1.330 | 0.611% | 0.0374%Vol |
| Span (β) | Consistency | Specific surface area | Surface area-average particle size D [3,2] |
| 1.998 | 0.62 | 0.284 $m^2/g$ | 21.149 μm |
| Volume-average particle size D[4,3] | $d_{(0.1)}$ | $d_{(0.5)}$ | $d_{(0.9)}$ |
| 32.364 μm | 11.066 μm | 25.939 μm | 62.903 μm |

Determination results show that the ultraviolet absorber in this example has particle size distribution characteristics as shown in Table 8 below, and a specific distribution curve is as shown in FIG. 4.

**Table 8 Particle size distribution table of an ultraviolet absorber in Example 4**

| Particle size (μm) | Volume within a range % | Particle size (μm) | Volume within a range % | Particle size (μm) | Volume within a range % |
|---|---|---|---|---|---|
| 0.724 | 0 | 5.754 | 0.39 | 45.709 | 6.15 |

(continued)

| Particle size (μm) | Volume within a range % | Particle size (μm) | Volume within a range % | Particle size (μm) | Volume within a range % |
|---|---|---|---|---|---|
| 0.832 | 0 | 6.607 | 0.74 | 52.481 | 5.37 |
| 0.955 | 0 | 7.586 | 1.26 | 60.256 | 4.53 |
| 1.096 | 0 | 8.710 | 1.96 | 69.183 | 3.67 |
| 1.259 | 0 | 10.000 | 2.79 | 79.433 | 2.84 |
| 1.445 | 0 | 11.482 | 3.75 | 91.201 | 2.06 |
| 1.660 | 0 | 13.183 | 4.74 | 104.713 | 1.37 |
| 1.905 | 0 | 15.136 | 5.71 | 120.226 | 0.8 |
| 2.188 | 0 | 17.378 | 6.55 | 138.038 | 0.4 |
| 2.512 | 0 | 19.953 | 7.22 | 158.489 | 0.1 |
| 2.884 | 0 | 22.909 | 7.65 | 181.970 | 0 |
| 3.311 | 0 | 26.303 | 7.81 | 208.930 | 0 |
| 3.802 | 0 | 30.200 | 7.71 | 239.883 | 0 |
| 4.365 | 0.05 | 34.674 | 7.37 | 275.423 | 0 |
| 5.012 | 0.16 | 39.811 | 6.84 | 316.228 | 0 |

The ultraviolet absorber in this example has an $\alpha_{d5}$ value of 0.002, an $\alpha_{d10}$ value of 0.074, and a difference between D[3,2] and D[4,3] being 11.215 μm.

## Example 5

The ultraviolet absorber in this example is determined by a laser diffraction method, a Malvern MS2000 laser particle size analyzer is used as a particle size analyzer, and determination conditions and test results are as shown in Table 9 below.

**Table 9 Particle size determination data in Example 5**

| Refractive index of particles | Absorptivity of particles | Particle size range | Shading degree |
|---|---|---|---|
| 1.590 | 0 | 0.020-2000 μm | 18.60% |
| Name of a dispersant | Refractive index of a dispersant | Residual error | Concentration |
| Water | 1.330 | 0.495% | 0.0575%Vol |
| Span (β) | Consistency | Specific surface area | Surface area-average particle size D [3,2] |
| 1.944 | 0.596 | 0.281 $m^2/g$ | 21.390 μm |
| Volume-average particle size D[4,3] | $d_{(0.1)}$ | $d_{(0.5)}$ | $d_{(0.9)}$ |
| 43.271 μm | 10.747 μm | 21.390 μm | 43.271 μm |

Determination results show that the ultraviolet absorber in this example has particle size distribution characteristics as shown in Table 10 below, and a specific distribution curve is as shown in FIG. 5.

**Table 10 Particle size distribution table of an ultraviolet absorber in Example 5**

| Particle size (μm) | Volume within a range % | Particle size (μm) | Volume within a range % | Particle size (μm) | Volume within a range % |
|---|---|---|---|---|---|
| 0.724 | 0 | 5.754 | 0.84 | 45.709 | 7.70 |
| 0.832 | 0 | 6.607 | 0.95 | 52.481 | 7.66 |
| 0.955 | 0 | 7.586 | 1.08 | 60.256 | 7.30 |
| 1.096 | 0 | 8.710 | 1.25 | 69.183 | 6.61 |

(continued)

| Particle size (μm) | Volume within a range % | Particle size (μm) | Volume within a range % | Particle size (μm) | Volume within a range % |
|---|---|---|---|---|---|
| 1.259 | 0 | 10.000 | 1.46 | 79.433 | 5.67 |
| 1.445 | 0.01 | 11.482 | 1.75 | 91.201 | 4.55 |
| 1.660 | 0.08 | 13.183 | 2.11 | 104.713 | 3.37 |
| 1.905 | 0.15 | 15.136 | 2.58 | 120.226 | 2.22 |
| 2.188 | 0.23 | 17.378 | 3.16 | 138.038 | 1.27 |
| 2.512 | 0.30 | 19.953 | 3.84 | 158.489 | 0.43 |
| 2.884 | 0.38 | 22.909 | 4.61 | 181.970 | 0.05 |
| 3.311 | 0.46 | 26.303 | 5.42 | 208.930 | 0 |
| 3.802 | 0.55 | 30.200 | 6.21 | 239.883 | 0 |
| 4.365 | 0.64 | 34.674 | 6.91 | 275.423 | 7.70 |
| 5.012 | 0.73 | 39.811 | 7.43 | 316.228 | 7.66 |

The ultraviolet absorber in this example has an $\alpha_{d5}$ value of 0.037, an $\alpha_{d10}$ value of 0.095, and a difference between D[3,2] and D[4,3] being 21.881 μm.

**Comparative Example 1**

In this comparative example, a DHHB product with a trade name of Uvinul A Plus, manufactured by BASF, is used as a sample. The sample in this comparative example is determined by a laser diffraction method, a Malvern MS2000 laser particle size analyzer is used as a particle size analyzer, and determination conditions and test results are as shown in Table 11 below.

**Table 11 Particle size determination data in Comparative Example 1**

| Refractive index of particles | Absorptivity of particles | Particle size range | Shading degree |
|---|---|---|---|
| 1.590 | 0 | 0.020-2000 μm | 15.74% |
| Name of a dispersant | Refractive index of a dispersant | Residual error | Concentration |
| Water | 1.330 | 1.097% | 0.0500%Vol |
| Span (β) | Consistency | Specific surface area | Surface area-average particle size D[3,2] |
| 10.995 | 2.44 | 0.258 $m^2/g$ | 23.263 μm |
| Volume-average particle size D[4,3] | $d_{(0.1)}$ | $d_{(0.5)}$ | $d_{(0.9)}$ |
| 151.879 μm | 10.517 μm | 51.970 μm | 518.950 μm |

Determination results show that the ultraviolet absorber in this comparative example has particle size distribution characteristics as shown in Table 12 below, and a specific distribution curve is as shown in FIG. 6.

**Table 12 Particle size distribution table of an ultraviolet absorber in Comparative Example 1**

| Particle size (μm) | Volume within a range % | Particle size (μm) | Volume within a range % | Particle size (μm) | Volume within a range % | Particle size (μm) | Volume within a range % |
|---|---|---|---|---|---|---|---|
| 0.724 | 0 | 5.754 | 0.81 | 45.709 | 5.23 | 363.078 | 0 |
| 0.832 | 0 | 6.607 | 0.93 | 52.481 | 5.58 | 416.869 | 0.18 |
| 0.955 | 0 | 7.586 | 1.07 | 60.256 | 5.79 | 478.630 | 0.63 |
| 1.096 | 0 | 8.710 | 1.24 | 69.183 | 5.82 | 549.541 | 1.08 |
| 1.259 | 0.02 | 10.000 | 1.42 | 79.433 | 5.63 | 630.957 | 1.48 |

(continued)

| Particle size (μm) | Volume within a range % | Particle size (μm) | Volume within a range % | Particle size (μm) | Volume within a range % | Particle size (μm) | Volume within a range % |
|---|---|---|---|---|---|---|---|
| 1.445 | 0.10 | 11.482 | 1.62 | 91.201 | 5.20 | 724.436 | 1.75 |
| 1.660 | 0.18 | 13.183 | 1.85 | 104.713 | 4.56 | 831.764 | 1.83 |
| 1.905 | 0.24 | 15.136 | 2.10 | 120.226 | 3.75 | 954.993 | 1.72 |
| 2.188 | 0.30 | 17.378 | 2.38 | 138.038 | 2.85 | 1096.478 | 1.44 |
| 2.512 | 0.36 | 19.953 | 2.69 | 158.489 | 1.95 | 1258.925 | 1.07 |
| 2.884 | 0.42 | 22.909 | 3.05 | 181.970 | 1.15 | 1445.440 | 0.69 |
| 3.311 | 0.48 | 26.303 | 3.45 | 208.930 | 0.46 | 1659.587 | 0.39 |
| 3.802 | 0.54 | 30.200 | 3.88 | 239.883 | 0 | 1905.461 | 0.15 |
| 4.365 | 0.62 | 34.674 | 4.34 | 275.423 | 0 | 2187.762 | 0.04 |
| 5.012 | 0.71 | 39.811 | 4.80 | 316.228 | 0 | | |

The ultraviolet absorber in this comparative example has an $\alpha_{d5}$ value of 0.005, an $\alpha_{d10}$ value of 0.122, and a difference between D[3,2] and D[4,3] being 128.616 μm.

**First dissolution experiment**

In this dissolution experiment, the agglomeration degree of the ultraviolet absorbers in the above examples and comparative examples was evaluated, and each sample of a certain mass was added into an oil phase and stirred at room temperature (25°C) at a stirring rate of 150 r/min for dissolution. The dissolution experiment was carried out on raw materials for preparation of a sunscreen oil with a sun protection factor (SPF) value of 25. The raw materials are as shown in Table 13.

**Table 13 Raw material table**

| Component | Content % |
|---|---|
| Octyl 4-methoxycinnamate (OMC) | 10 |
| Diethylamino hydroxybenzoyl hexyl benzoate (DHHB) | 3 |
| 2-ethylhexyl salicylate ($C_{15}H_{22}O_3$) (OS) | 5 |
| Dicaprylyl carbonate ($C_{17}H_{34}O_3$) | 10 |
| Dibutyl adipate ($C_{14}H_{26}O_4$) | 15 |
| Caprylic/capric triglyceride ($C_{24}H_{48}O_8$) | 56.5 |
| Tocopheryl acetate ($C_{31}H_{52}O_3$) | 0.5 |

Except that DHHB is a solid, all other components are liquid oils. A production process includes: sequentially adding various components into an oil pot, and enabling a stirring function until the components are completely dissolved. As for the above raw materials and the process, laboratory simulation test results are as shown in Table 14 below.

**Table 14 Sample property test result table**

| Sample name | Span (β) | Particle size distribution $d_{(0.1)}$, $d_{(0.9)}$ (integer) | $\alpha_{d10}$ | Sample dissolution time |
|---|---|---|---|---|
| Example 1 | 2.008 | 10,58 | 0.111 | < 5 min |
| Example 2 | 1.869 | 10, 52 | 0.104 | < 5 min |
| Example 3 | 2.23 | 10,78 | 0.107 | < 5 min |
| Example 4 | 1.998 | 11, 63 | 0.074 | < 5 min |
| Example 5 | 1.944 | 10, 43 | 0.095 | < 5 min |
| Comparative Example 1 | 10.995 | 11,519 | 0.122 | 151 min |
| Comparative Example 2 | | 850 (±20), 1000 | | 134 min |

According to the data in Table 14:
in combination with FIG. 7, it can be seen that when the samples are stirred at an added concentration of 3%, a temperature of 25°C and a stirring rate of 150 r/min for dissolution, the dissolution time of DHHB ultraviolet absorbers provided in Examples 1-5 of the present disclosure is shorter than 5 min.

In combination with FIG. 8 to FIG. 10, it can be seen that under same dissolution conditions, the sample in Comparative Example 1 still contains obvious undissolved DHHB particles after dissolved for 5 min and 45 min and is finally dissolved completely for 151 min.

In combination with Comparative Example 1 and Comparative Example 2, it can be seen that under the influence of various actual factors, the dissolution rate of DHHB ultraviolet absorber is not necessarily consistent with a traditional rule that particles with a smaller particle size have a higher dissolution rate.

**Second dissolution experiment**

In this dissolution experiment, the agglomeration degree of the ultraviolet absorbers in the above examples and comparative examples was evaluated, and each sample of a certain mass was added into an oil phase and stirred at room temperature (25°C) at a stirring rate of 150 r/min for dissolution. The dissolution experiment was carried out on raw materials for preparation of a sunscreen oil with an SPF value of 25. The raw materials are as shown in Table 15.

**Table 15 Raw material table**

| Component | Content % |
|---|---|
| Octyl 4-methoxycinnamate (OMC) | 10 |
| Diethylamino hydroxybenzoyl hexyl benzoate (DHHB) | 6 |
| 2-ethylhexyl salicylate ($C_{15}H_{22}O_3$) (OS) | 5 |
| Dicaprylyl carbonate ($C_{17}H_{34}O_3$) | 10 |
| Dibutyl adipate ($C_{14}H_{26}O_4$) | 15 |
| Caprylic/capric triglyceride ($C_{24}H_{48}O_8$) | 53.5 |
| Tocopheryl acetate ($C_{31}H_{52}O_3$) | 0.5 |

Except that DHHB is a solid, all other components are liquid oils. A production process includes: sequentially adding various components into an oil pot, and enabling a stirring function until the components are completely dissolved. As for the above raw materials and the process, laboratory simulation test results are as shown in Table 16 below.

**Table 16 Sample property test result table**

| Sample name | Span (β) | Particle size distribution $d_{(0.1)}$- $d_{(0.9)}$ (integer) | $\alpha_{d10}$ | Sample dissolution time |
|---|---|---|---|---|
| Example 1 | 2.008 | 10-58 | 0.111 | < 5 min |
| Example 2 | 1.869 | 10-52 | 0.104 | < 5 min |
| Example 3 | 2.23 | 10-78 | 0.107 | < 5 min |
| Example 4 | 1.998 | 11-63 | 0.074 | < 5 min |
| Example 5 | 1.944 | 10-43 | 0.095 | < 5 min |
| Comparative Example 1 | 10.995 | 11-519 | 0.122 | 208 min |
| Comparative Example 2 | | 850 (±20)-1000 | | 202 min |

From Table 16, it can be seen that:
when the added concentration is changed to 6% and the temperature and the stirring rate are the same as those in the first dissolution experiment, the dissolution time of DHHB ultraviolet absorbers provided in Examples 1-5 of the present disclosure is also shorter than 5 min, indicating that the dissolution time is not affected by the added concentration.

However, through comparison between Table 14 and Table 16, it can be seen that the dissolution time of the sample in Comparative Example 1 is increased from 151 min to 208 min at an increase rate of about 38%, and the dissolution time of the sample in Comparative Example 2 is increased from 134 min to 202 min at an increase rate of about 50%, indicating that the dissolution time of the two samples is affected by the added concentration and is greatly increased.

In previous production technologies, studies are mostly focused on optimization and regulation of a method or an apparatus of a single link or other aspects, so as to achieve the purpose of optimizing an entire production process, and in fact, some effects have been achieved. However, a relatively big problem restricting optimization of the production process is still present, that is, the stability of a production rhythm, which has been rarely focused by researchers. A production process with a stable rhythm requires each procedure to perform production strictly in accordance with a certain rhythm. When the production rhythm of a certain procedure is unstable, the smoothness of the entire production process is affected. The production smoothness not only affects the production efficiency of a production technology, but also directly affects the product quality in a production process. Unsmooth operation of a technology in the field of cosmetic products often directly leads to other production quality problems, and finally, the stability of a product is affected.

As shown above, an actual process of using DHHB particles to produce sunscreen products involves many links, and exemplary simulation tests listed in the first and second dissolution experiments are only one link in a production process. Moreover, when DHHB particles of different models and concentrations are added in the link, the dissolution time is prone to change greatly, leading to serious deterioration of the stability of the production technology, seriously affecting the stability of the production rhythm, and further leading to poor stability of the product quality.

## Claims

1. An ultraviolet absorber, wherein

    the ultraviolet absorber is 2-(4-N,N-diethylamino-2-hydroxybenzoyl) hexyl benzoate (DHHB), specifically having a structural formula as follows:

    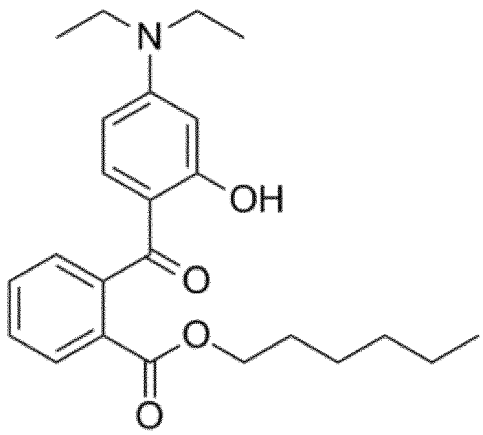

    the ultraviolet absorber has a median particle size $d_{(0.5)}$ of less than 100 $\mu$m;
    a maximum particle size $d_{(0.9)}$ of the ultraviolet absorber is within a range of 50-100 $\mu$m; and
    a width difference between a minimum particle size $d_{(0.1)}$ and a maximum particle size $d_{(0.9)}$ of the ultraviolet absorber is within a range of 30-100 $\mu$m.

2. The ultraviolet absorber according to claim 1, wherein the maximum particle size $d_{(0.9)}$ is within a range of 50-90 $\mu$m.

3. The ultraviolet absorber according to claim 1, wherein the maximum particle size $d_{(0.9)}$ is within a range of 50-80 $\mu$m.

4. The ultraviolet absorber according to claim 1, wherein the width difference between the minimum particle size $d_{(0.1)}$ and the maximum particle size $d_{(0.9)}$ is within a range of 30-70 $\mu$m.

5. The ultraviolet absorber according to claim 1, wherein the width difference between the minimum particle size $d_{(0.1)}$ and the maximum particle size $d_{(0.9)}$ is within a range of 40-50 $\mu$m.

6. The ultraviolet absorber according to claim 1, wherein the minimum particle size $d_{(0.1)}$ is within a range of 5-15 $\mu$m.

7. The ultraviolet absorber according to any one of claims 1-6, wherein the ultraviolet absorber has an effective span of not greater than 3 as determined by an instrument.

8. The ultraviolet absorber according to claim 7, wherein the ultraviolet absorber has a consistency within a range of 0.5-1.5 as determined by an instrument.

9. The ultraviolet absorber according to claim 7, wherein the ultraviolet absorber has a consistency within a range of 0.5-1 as determined by an instrument.

10. The ultraviolet absorber according to claim 7, wherein the median particle size $d_{(0.5)}$ of the ultraviolet absorber is within a range of 20-40 $\mu$m.

11. The ultraviolet absorber according to claim 1, wherein a particle size distribution of the ultraviolet absorber meets the following requirements:

$$\alpha_{d10} = \frac{V_{d10}}{V_{d100}}$$

wherein

$\alpha_{d10}$ is a distribution coefficient of 10 $\mu$m particles;
$V_{d10}$ is a total volume ratio of particles with a particle size of less than 10 $\mu$m in the ultraviolet absorber to the ultraviolet absorber particles, %;
$V_{d100}$ is a total volume ratio of particles with a particle size of less than 100 $\mu$m in the ultraviolet absorber to the ultraviolet absorber particles, %;
and a distribution coefficient of small particle size particles of the ultraviolet absorber satisfies that $\alpha_{d10}$ is equal to or less than 0.2.

12. The ultraviolet absorber according to claim 11, wherein the distribution coefficient of small particle size particles satisfies that $\alpha_{d10}$ is equal to or less than 0.15.

13. The ultraviolet absorber according to claim 11, wherein the distribution coefficient of small particle size particles satisfies that $\alpha_{d10}$ is equal to or greater than 0.05 and equal to or less than 0.15; and preferably, the distribution coefficient of small particle size particles satisfies that $\alpha_{d10}$ is equal to or greater than 0.07 and equal to or less than 0.12.

14. The ultraviolet absorber according to any one of claims 11-13, wherein $V_{d100}$ of the ultraviolet absorber is not less than 70%, preferably, not less than 85%, and further preferably, not less than 95%.

15. The ultraviolet absorber according to any one of claims 1-6 or claims 11-13, wherein under processing conditions, a dissolution time of the ultraviolet absorber in a solvent is not higher than 30 min;
the processing conditions are specifically as follows:

solvent: dibutyl adipate;
added concentration: 3-20 wt%;
temperature: 20-30°C;
stirring rate: 150±5 r/min;
and an index of the ultraviolet absorber dissolved in the solvent is that the solvent has a solid content of less than 0.5%.

16. The ultraviolet absorber according to claim 15, wherein the dissolution time of the ultraviolet absorber in a solvent is not higher than 25 min.

17. A composition having an ultraviolet absorption effect, comprising: an ultraviolet absorber, a dispersant, a film forming agent, an antioxidant and a lubricant; wherein

the ultraviolet absorber comprises the ultraviolet absorber according to any one of claims 1-16;
and at least one of the dispersant, the film forming agent, the antioxidant and the lubricant is an oil phase at a temperature of not lower than 20°C.

**18.** The composition having an ultraviolet absorption effect according to claim 17, comprising: the 2-(4-N,N-diethylamino-2-hydroxybenzoyl) hexyl benzoate ultraviolet absorber particles according to any one of claims 1-16, dicaprylyl carbonate, dibutyl adipate, caprylic/capric triglyceride and tocopheryl acetate.

**19.** The ultraviolet absorber according to any one of claims 1-16 or the composition according to any one of claims 17-18 for use as a sunscreen component of a cosmetic product, or as an ultraviolet protection additive component for fabric surface treatment, or as an ultraviolet protection additive component for a polymer material.

**20.** A cosmetic product, comprising the ultraviolet absorber according to any one of claims 1-16 or comprising the composition according to any one of claims 17-18 as a raw material.

**21.** A cosmetic product preparation process, wherein the ultraviolet absorber according to any one of claims 1-16 or the composition according to any one claims 17-18 is used as a raw material, and when the raw material is dissolved in an oil phase:

a temperature is not higher than 50°C, and is preferably 40-50°C;
and a concentration added is not higher than 20 wt%, and is preferably 3-20 wt%.

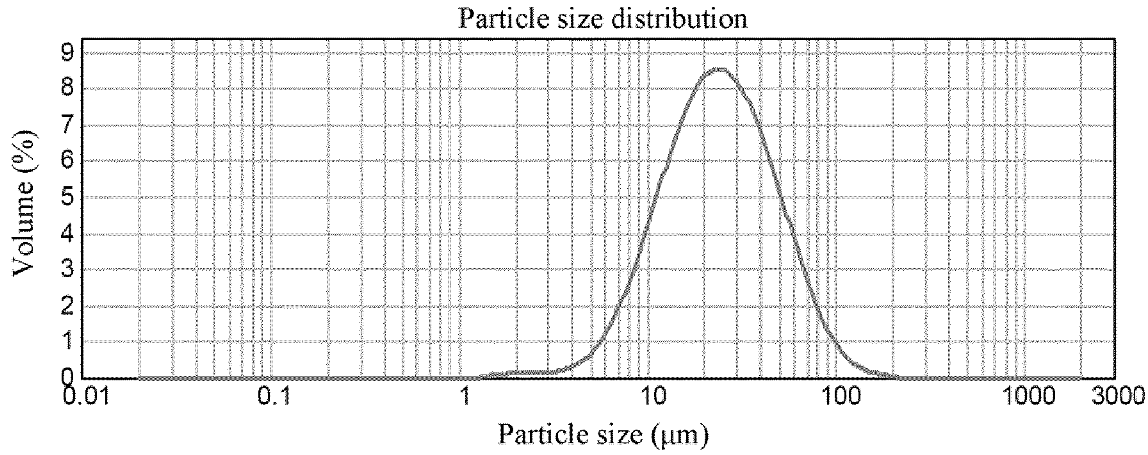


FIG. 1

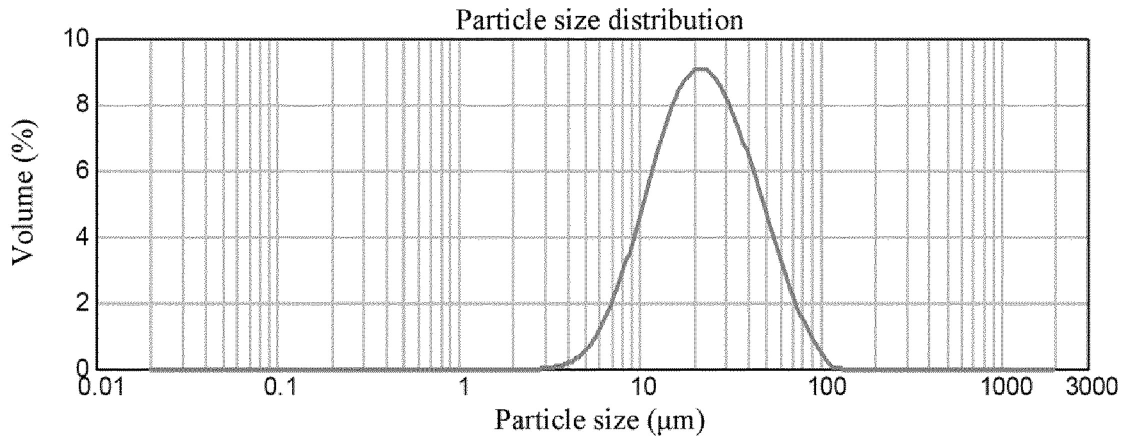


FIG. 2

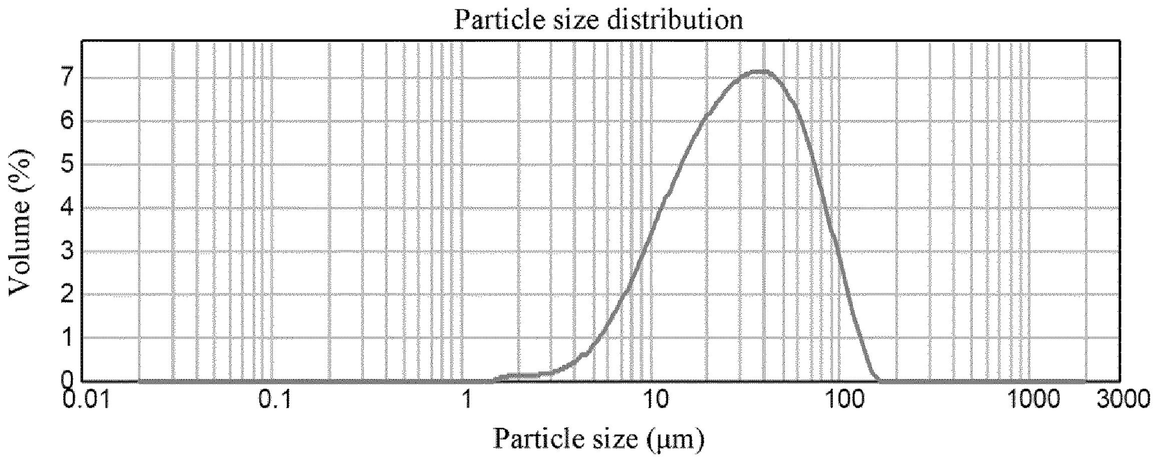


FIG. 3

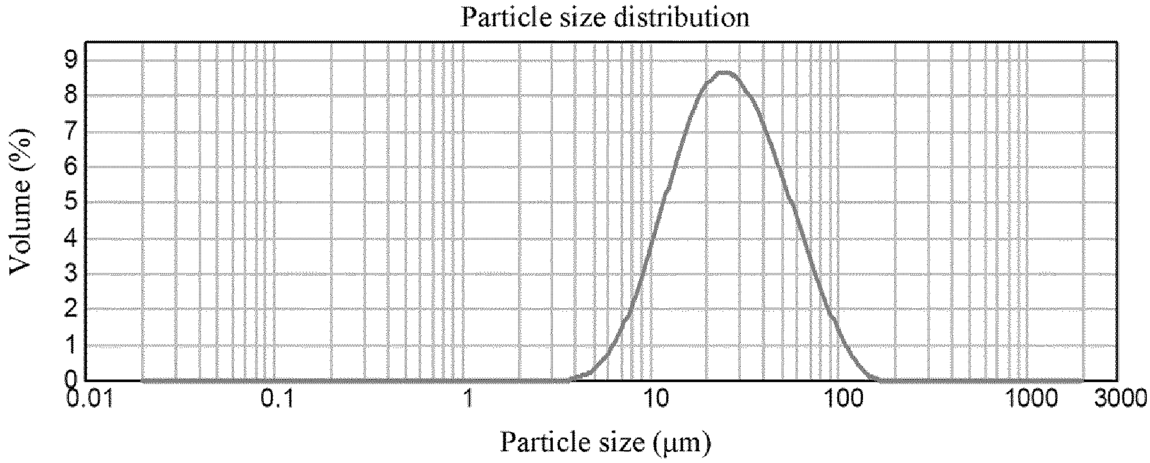


FIG. 4

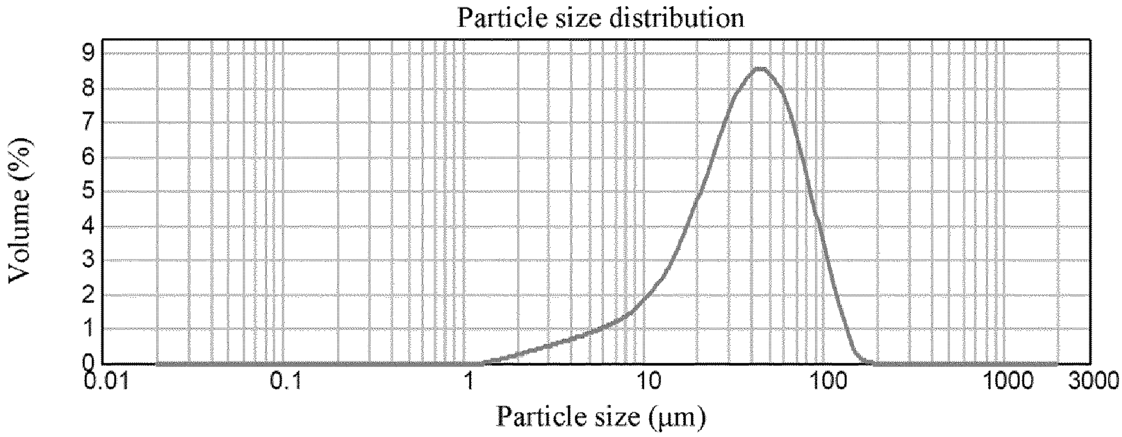


FIG. 5

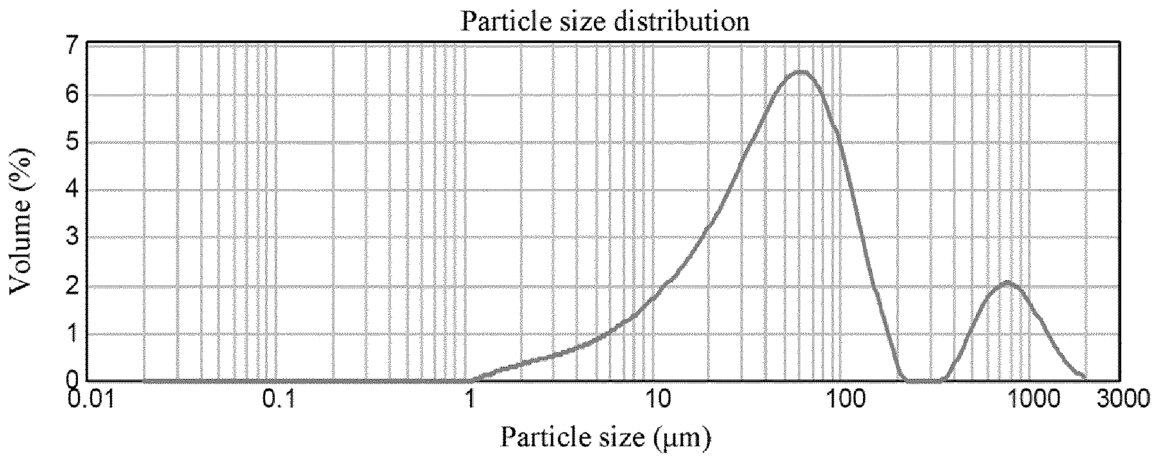


FIG. 6

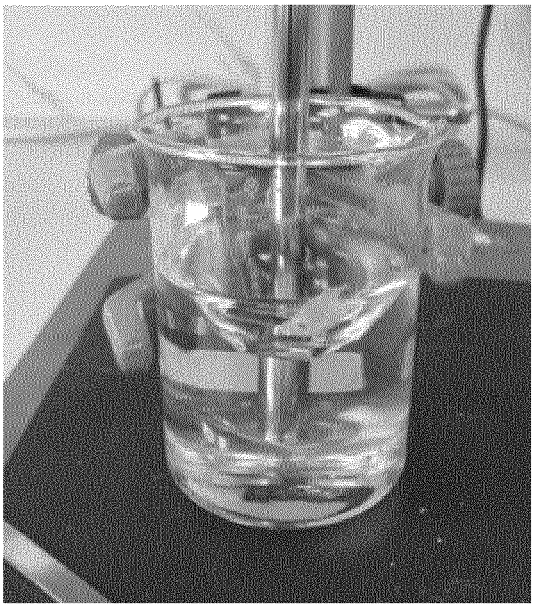

FIG. 7

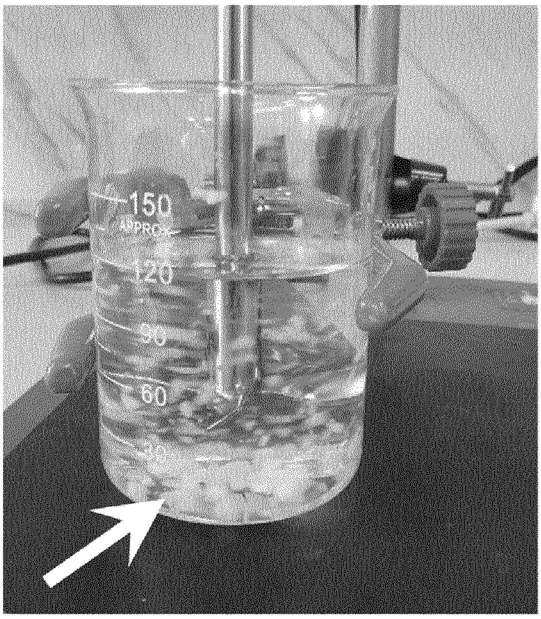


FIG. 8

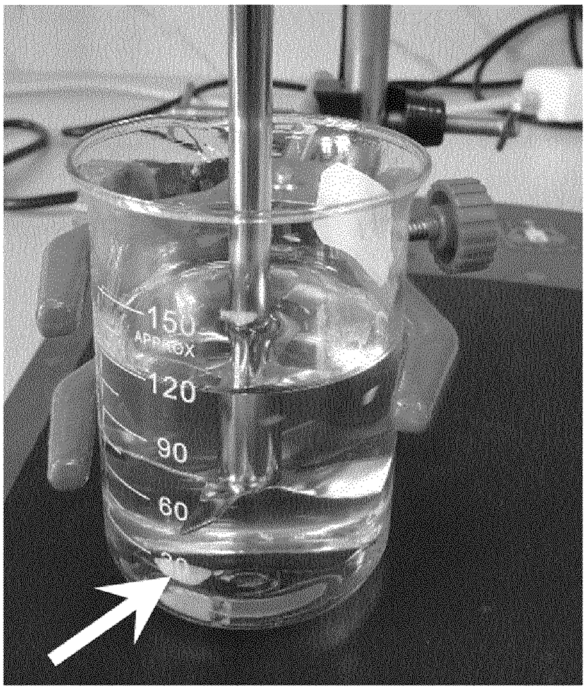


FIG. 9

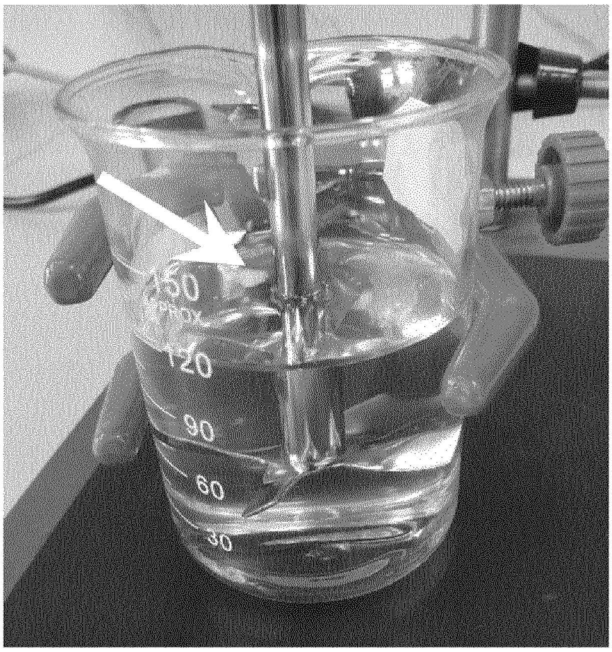


FIG. 10

**INTERNATIONAL SEARCH REPORT**

International application No. **PCT/CN2022/142177**

**A. CLASSIFICATION OF SUBJECT MATTER**

C07C 229/52(2006.01)i;D06M 13/372(2006.01)i;C08K 5/18(2006.01)i;A61K 8/41(2006.01)i;A61Q 17/04(2006.01)i

According to International Patent Classification (IPC) or to both national classification and IPC

**B. FIELDS SEARCHED**

Minimum documentation searched (classification system followed by classification symbols)

C07C; D06M; C08K; A61K; A61Q

Documentation searched other than minimum documentation to the extent that such documents are included in the fields searched

Electronic data base consulted during the international search (name of data base and, where practicable, search terms used)

CNTXT; ENTXT; WPABS; DWPI; VEN; CNKI; STN(CAPLUS, REGISTRY, MARPAT): 紫外线吸收剂, 粒度, 粒径, ultraviolet, absorber, width, structural formula search

**C. DOCUMENTS CONSIDERED TO BE RELEVANT**

| Category* | Citation of document, with indication, where appropriate, of the relevant passages | Relevant to claim No. |
|---|---|---|
| X | TW 202122369 A (ASTECH CO., LTD.) 16 June 2021 (2021-06-16) entire document, in particular claim 8, and embodiments 1-9 | 1-21 |
| X | US 2010015070 A1 (BASF SE) 21 January 2010 (2010-01-21) entire document, in particular example 35, and description, page 54, right column, the third formula | 17-21 |
| X | DE 102007035567 A1 (BASF SE; MERCK PATENT GMBH;) 29 January 2009 (2009-01-29) entire document, and in particular embodiments | 17-21 |
| X | WO 2010043588 A1 (BASF SE et al.) 22 April 2010 (2010-04-22) entire document, and in particular embodiments | 17-21 |
| X | CN 101365493 A (BASF AG) 11 February 2009 (2009-02-11) entire document, and in particular embodiments | 17-21 |
| X | US 2008247975 A1 (DUEVA-KOGANOV OLGA V et al.) 09 October 2008 (2008-10-09) entire document, and in particular embodiments | 17-21 |
| X | CN 101365416 A (BASF AG) 11 February 2009 (2009-02-11) entire document, and in particular embodiments | 17-21 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **01 March 2023** | **06 March 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No.
**PCT/CN2022/142177**

**C. DOCUMENTS CONSIDERED TO BE RELEVANT**

| Category* | Citation of document, with indication, where appropriate, of the relevant passages | Relevant to claim No. |
|---|---|---|
| A | CN 101675029 A (BASF AG) 17 March 2010 (2010-03-17)<br>entire document | 1-21 |
| A | CN 1653035 A (BASF AG) 10 August 2005 (2005-08-10)<br>entire document | 1-21 |
| A | CN 101378808 A (CIBA SC HOLDING AG) 04 March 2009 (2009-03-04)<br>entire document | 1-21 |
| A | CN 109908020 A (THE MENTHOLATUM (CHINA) PHARMACEUTICALS CO., LTD.) 21 June 2019 (2019-06-21)<br>entire document | 1-21 |
| A | WO 2021170695 A1 (BASF AG) 02 September 2021 (2021-09-02)<br>entire document | 1-21 |
| A | CN 105358221 A (BASF AG) 24 February 2016 (2016-02-24)<br>entire document | 1-21 |
| A | CN 111150677 A (GUANGZHOU WEIHONGQI BIOTECHNOLOGY CO., LTD.) 15 May 2020 (2020-05-15)<br>entire document | 1-21 |

Form PCT/ISA/210 (second sheet) (July 2022)

**INTERNATIONAL SEARCH REPORT**
**Information on patent family members**

International application No.
**PCT/CN2022/142177**

| Patent document cited in search report | Publication date (day/month/year) | Patent family member(s) | Publication date (day/month/year) |
|---|---|---|---|
| TW 202122369 A | 16 June 2021 | DE 112020004927 T5 | 23 June 2022 |
| | | WO 2021071033 A1 | 15 April 2021 |
| | | WO 2021071033 A9 | 14 October 2021 |
| | | JP 2022188148 A | 20 December 2022 |
| | | KR 20210142575 A | 25 November 2021 |
| | | KR 102439837 B1 | 05 September 2022 |
| | | JP 2021533088 A | 02 December 2021 |
| | | JP 7195351 B2 | 23 December 2022 |
| | | KR 20200002720 A | 08 January 2020 |
| | | KR 102179649 B1 | 18 November 2020 |
| | | JP 2022188149 A | 20 December 2022 |
| | | KR 20210071804 A | 16 June 2021 |
| | | KR 102357197 B1 | 03 February 2022 |
| US 2010015070 A1 | 21 January 2010 | JP 2010509279 A | 25 March 2010 |
| | | CA 2664033 A1 | 15 May 2008 |
| | | EP 2066691 A1 | 10 June 2009 |
| | | WO 2008055931 A1 | 15 May 2008 |
| DE 102007035567 A1 | 29 January 2009 | None | |
| WO 2010043588 A1 | 22 April 2010 | None | |
| CN 101365493 A | 11 February 2009 | ZA 200805382 A | 30 December 2009 |
| | | ZA 200805381 A | 25 November 2009 |
| US 2008247975 A1 | 09 October 2008 | BRPI 0809925 A2 | 23 September 2014 |
| | | EP 2129363 A2 | 09 December 2009 |
| | | WO 2008122516 A2 | 16 October 2008 |
| | | WO 2008122516 A3 | 05 March 2009 |
| | | KR 20090127193 A | 09 December 2009 |
| | | JP 2010523519 A | 15 July 2010 |
| | | MX 2009010535 A | 22 October 2009 |
| CN 101365416 A | 11 February 2009 | None | |
| CN 101675029 A | 17 March 2010 | RU 2009144285 A | 10 June 2011 |
| | | RU 2487114 C2 | 10 July 2013 |
| | | BRPI 0810872 A2 | 29 October 2014 |
| | | BRPI 0810872 B1 | 19 July 2022 |
| | | US 2014349115 A1 | 27 November 2014 |
| | | US 9550727 B2 | 24 January 2017 |
| | | US 2010137629 A1 | 03 June 2010 |
| | | US 8802887 B2 | 12 August 2014 |
| | | WO 2008135360 A1 | 13 November 2008 |
| | | KR 20100017539 A | 16 February 2010 |
| | | KR 101486606 B1 | 27 January 2015 |
| | | ES 2402170 T3 | 29 April 2013 |
| | | AU 2008248760 A1 | 13 November 2008 |
| | | CA 2683483 A1 | 13 November 2008 |
| | | JP 2010525009 A | 22 July 2010 |
| | | JP 5450384 B2 | 26 March 2014 |
| | | EP 2155660 A1 | 24 February 2010 |
| | | EP 2155660 B1 | 27 February 2013 |
| CN 1653035 A | 10 August 2005 | PT 1506159 E | 29 October 2007 |
| | | US 2005165099 A1 | 28 July 2005 |

Form PCT/ISA/210 (patent family annex) (July 2022)

**INTERNATIONAL SEARCH REPORT**
**Information on patent family members**

International application No.
**PCT/CN2022/142177**

| Patent document cited in search report | | | Publication date (day/month/year) | Patent family member(s) | | | Publication date (day/month/year) |
|---|---|---|---|---|---|---|---|
| | | | | AU | 2003224156 | A1 | 02 December 2003 |
| | | | | ES | 2290452 | T3 | 16 February 2008 |
| | | | | EP | 1506159 | A1 | 16 February 2005 |
| | | | | EP | 1506159 | B1 | 19 September 2007 |
| | | | | DK | 1506159 | T3 | 27 December 2007 |
| | | | | JP | 2005533764 | A | 10 November 2005 |
| | | | | JP | 4472518 | B2 | 02 June 2010 |
| | | | | DE | 10221805 | A1 | 27 November 2003 |
| | | | | WO | 03097578 | A1 | 27 November 2003 |
| | | | | AT | 373634 | T | 15 October 2007 |
| | | | | DE | 50308226 | D1 | 31 October 2007 |
| CN | 101378808 | A | 04 March 2009 | | None | | |
| CN | 109908020 | A | 21 June 2019 | | None | | |
| WO | 2021170695 | A1 | 02 September 2021 | BR | 112022016712 | A2 | 11 October 2022 |
| | | | | KR | 20220147622 | A | 03 November 2022 |
| | | | | EP | 4110754 | A1 | 04 January 2023 |
| | | | | TW | 202140416 | A | 01 November 2021 |
| CN | 105358221 | A | 24 February 2016 | PT | 2994201 | T | 22 March 2018 |
| | | | | KR | 20160007573 | A | 20 January 2016 |
| | | | | KR | 102239036 | B1 | 13 April 2021 |
| | | | | WO | 2014180818 | A1 | 13 November 2014 |
| | | | | BR | 112015028140 | A2 | 25 July 2017 |
| | | | | BR | 112015028140 | B1 | 13 October 2020 |
| | | | | ES | 2663478 | T3 | 12 April 2018 |
| | | | | EP | 2994201 | A1 | 16 March 2016 |
| | | | | EP | 2994201 | B1 | 20 December 2017 |
| | | | | RU | 2015152831 | A | 16 June 2017 |
| | | | | RU | 2677885 | C2 | 22 January 2019 |
| | | | | JP | 2016517898 | A | 20 June 2016 |
| | | | | JP | 6462667 | B2 | 30 January 2019 |
| | | | | US | 2016067158 | A1 | 10 March 2016 |
| CN | 111150677 | A | 15 May 2020 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 105358221 B
- CN 109908020 A